# EUROPEAN PATENT APPLICATION

(11) **EP 4 684 727 A1**
(43) Date of publication of application: **28.01.2026**
(21) Application number: 24382813.4
(22) Date of filing: 24.07.2024
(51) Int. Cl.: A61B 5/103, A61B 5/00

(54) **PRESSURE SENSING FILM, MANUFACTURE METHOD, USE AND PRESSURE ANALYSIS SYSTEM**

(71) Applicant: Fundació Eurecat, 08290 Cerdanyola del Vallès (ES)
(72) Inventor: Alique Garcia, Marc, 08302 Mataró (ES); Moya Lara, Ana, 08208 Sabadell (ES); Delgado Simao, Claudia Custodia, 08014 Barcelona (ES); Otero Chávez, David, 08302 Mataró (ES); Lacharmoise, Paul Dominique, 08041 Barcelona (ES); Plantà Torralba, Francisco Javier, 08320 El Masnou (ES); Catasús Capellades, Xavier, 08012 Barcelona (ES)
(74) Representative: Curell Suñol S.L.P.

(57) **Abstract**

Pressure sensing film (1), manufacture method, use and pressure analysis system. The film (1) having a sensing part (2) configured for being positioned between a supporting surface and a part of a user, and a connection part (3) in a usage position; the film (1) comprising:
- an elastic stretchable substrate layer (4);
- a plurality of pressure sensors (100) formed over said substrate layer (4) and having a capacitor-like structure with a piezoelectric pad (61) arranged between polymeric electrically conductive pads (51, 71);
- polymeric electrically conductive lines (52, 62) connecting the pressures sensors (100);
- a third conductive layer (8), connecting said conductive lines (52, 62) with terminal ends (83) arranged in the connection part (3); and
- an elastic stretchable dielectric layer (9) covering all the underlying layers.

## Description

### Field of the invention

The invention lies in the field of analysis of pressure distribution of a part of a user that is supported by an element having supporting surface. A particular and non-limiting example lies in the field of podiatry, for helping in the design and/or adaptation of customized insoles aimed for compensating or correcting the way a customer threads.

In particular, the invention relates to a pressure sensing film having a sensing part and a connection part, said sensing part configured for being positioned between a supporting surface and a part of a user in a usage position.

The invention also relates to a method for manufacturing a pressure sensing film.

The invention also relates to an insole pressure analysis system comprising a pressure sensing film, as well as to the usage of a pressure sensing film for obtaining a pressure map of a user over said particular supporting surface, for example, the thread of the user with a particular insole.

### Prior art

Therapeutical or orthopaedical insoles are commonly used for compensating the way a user threads on the shoe, for example, in order to avoid instabilities that could lead to discomfort and even to injuries. Some shoe manufacturers have created shoes especially adapted to compensate common issues for the so-called pronator or supinator users. This is commonly found in running shoes, but the degree of adaptation to the user is very limited.

Better results may be obtained by designing an insole for the specific user, for example, for a particular shoe. Podiatrists generally design the insole, which is latter tested with the user in position, wearing the shoe with the insole. However, the testing is often only qualitatively assessed, the podiatrist simply observes the user walking and determines if the insole must be corrected and how.

This type of testing and adaptation is not precise, the results may vary and may require several iterations of testing and adaptation. Therefore, it is needed a solution for providing better and, if possible, quantitative results when adapting an insole to a user.

As it will be discussed, the resulting invention may also be extended to other fields wherein the supporting surface is not an insole and the user part that is supported is not the foot.

### Summary of the invention

The invention is aimed to provide a pressure sensing film of the type stated at the beginning, being able to avoid the problems that have been identified above.

This purpose is achieved by a pressure sensing film of the type indicated at the beginning, characterized in that said pressure sensing film comprises:
- a substrate layer, made of an elastic stretchable material;
- a first conductive layer, arranged over said substrate layer;
- a piezoelectric layer, arranged over said first conductive layer;
- a second conductive layer, arranged over said piezoelectric layer;
- a third conductive layer, arranged over at least part of said second conductive layer; and
- a dielectric layer, made of an elastic stretchable dielectric material over said third conductive layer and covering all the underlying layers;
wherein said first conductive layer comprises a plurality of first conductive pads and one of more first conductive lines, all made of a first electrically conductive polymer, and arranged in said sensing part, such that each first conductive pad is connected to at least one of said first conductive lines, each first conductive line having a first terminal end; said piezoelectric layer comprises a plurality of piezoelectric pads made of a piezoelectric polymer, each piezoelectric pad arranged over one of said first conductive pads in said sensing part; said second conductive layer comprises a plurality of second conductive pads and one of more second conductive lines, all made of a second electrically conductive polymer, and arranged in said sensing part, such that each second conductive pad is connected to at least one of said second conductive lines, each second conductive line having a second terminal end, and wherein each second conductive pad is arranged over one of said piezoelectric pad, so that a plurality of pressure sensors having a capacitor-like structure is formed, each pressure sensor having a piezoelectric pad arranged between a first conductive pad and a second conductive pad; and said third conductive layer comprises a plurality of third electrically conductive lines made of an electrically conductive material, each third electrically conductive line having one third terminal end at said connection part and another end connected to one of said first terminal ends or one of said second terminal ends, said dielectric layer not covering said third terminal ends.

It may be envisaged embodiments wherein the film comprises one or more layers besides those stated hereinbefore. Unless otherwise specified, "arranged over" does not necessarily imply that the element is arranged directly over, it may be envisaged that intermediate elements are provided.

Thanks to the different layers and, in particular, the substrate and the dielectric layer, the pressure sensing film of the invention is elastic and stretchable. This has the advantage that the film adapts to the pressure of the part of the user that it is supported by the supporting surface. For example, in the case wherein the supporting surface is the insole of the shoe, the film adapts when the user threads on it. Indeed, a non-stretchable film may result in inaccurate pressure measurements because the film may move or reposition instead of stretching when the user threads over it and the insole and the foot expand.

The pressure sensing film comprises a plurality of pressure sensors arranged therein, each pressure sensor having a capacitor-like structure, that is, formed by a piezoelectric pad between two conductive pads. This structure allows that, when the piezoelectric material is compressed by the weight of the user, a potential difference is created between the first and second conductive pads. Moreover, the electrical capacity of each sensor may also be dependent on the pressure. Each of the two conductive pads of each pressure sensor is electrically connected to a corresponding line that, in turn, is electrically connected to the third terminal located in the connecting part of the film. The arrangement of the elements created by the different layers, and the use of electrically conductive polymers contribute to the general stretchability and elasticity of the pressure sensing film, thereby contributing to the technical objective of the device. Thanks to the third terminal ends arranged in the connection part of the film, the pressure sensing film can be connected to an external device for receiving the measurements of the plurality of sensors. Preferably, the connection part is arranged so that it lies behind the heel of the user in the case the supporting surface is an insole, so that it is easier to connect to any device located outside of the shoe. However, other embodiments may be envisaged wherein the connection part is provided in other positions.

Preferably, said supporting surface is an insole of a shoe and said part of the user is the sole of a foot of said user; or said supporting surface is a saddle and said part of the user are the buttocks of said user.

Preferably, said elastic stretchable material of said substrate layer is thermoplastic polyurethane, TPU. It has been found that this material has a good compatibility with several materials, in particular, with inks used when the manufacturing method is screen printing.

Preferably, said first conductive polymer is the same that said second conductive polymer, so that manufacturing is simplified and both layers have the same characteristics. More preferably, said polymer is poly(3,4-ethylenedioxythiophene) polystyrene sulfonate, usually known as PEDOT:PSS. This material has a good adhesion with elastic substrates and is compatible with the piezoelectric layer and other materials due to its low roughness profile and aqueous-based solvent, facilitating the development of multilayer structures, in particular, in capacitive devices. PEDOT:PSS can be processed to be compatible with large-scale fully screen printing manufacture. Moreover, the material is inherently flexible and can maintain its electrical properties even when bent or stretched. The material is also biocompatible and has a low cost.

Preferably, said piezoelectric polymer is poly(vinylidene fluoride-co-trifluoroethylene), usually known as PVDF-TrFE. This material has a high sensitivity to pressure changes, good thermal stability and biocompatibility. It also has a high dielectric constant, which improves efficiency and performance of capacity pressure sensors. The copolymer further has low electrical loss, which ensures that the energy consumption of the sensor is minimized, enhancing the overall efficiency of the device. PVDF-TrFE can be processed to be compatible with large-scale fully screen printing of the device. The combination of PVDF-TrFE with the PEDOT:PSS detailed above is particularly advantageous because the electron transfer charge with the PVDF-TrFE.

Preferably, said electrically conductive material of said third conductive layer is a metal, preferably silver. Many metals are good electrical conductors having a low electrical resistance; however, they are not typically stretchable or elastic. This drawback may be compensated by the fact that part of the electrically conductive lines (first and second conductive lines) are formed with a polymer and only part of them (third conductive lines), are made of metal, in particular, silver. This way, it is possible to take advantage of the good conductive characteristics of metals but keeping the film stretchable. This effect is particularly enhanced when the main part of third electrically conductive lines are arranged following ant of the lateral edges of said sensing part, and preferably near said edges, so that the central area of the sensing part of the film where all the pressure sensors are located is almost free of non-stretchable lines. It may be envisaged that the third electrical lines are between 3 and 10 mm from the edge of the sensing part. This is particularly advantageous in embodiment wherein the film is screen printed, because, even if flexible metals may exist, it is difficult to find metallic materials that are compatible with the sensor manufacturing structure so that all the elements are functional.

Preferably, at least one of said first conductive layer, said piezoelectric layer, said second conductive layer, said third conductive layer and said dielectric layer, and preferably all said layers, are formed by screen printing. Screen printing is particularly well suited for manufacturing the pressure sensing film of the invention because of the layered arrangement, the availability of different ink materials, and the precision that may be obtained by the screen printing method.

Preferably, said dielectric layer is formed by screen printing and said elastic stretchable dielectric material of said dielectric layer is a dielectric encapsulating ink, in particular, Dupond 8155 ink has been found to have a good balance regarding the electrical insulation, protection and stretchability.

Preferably, the pressure sensing film further comprises an adhesive layer arranged under said substrate layer, such that in said usage position, said adhesive layer is in contact with said supporting surface, for example, an insole, said adhesive layer preferably formed by screen printing or by spraying. The adhesive layer improves the positioning and fixation between the pressure sensing film and the supporting surface, making it less prone to move due to the movements of the user, for example, the foot. Preferably, the adhesive of the adhesive layer does not cover said third terminal ends and any said piezoelectric or conductive pads, in order to improve electrical connectivity and also to avoid dampening the pressure sensor signals.

In an alternative embodiment, the adhesive layer is arranged as a final step over the other layers, not covering the terminals, conductive lines, sensors, etc. So that it does not affect their function.

Preferably, said piezoelectric pads are larger than said first conductive pads and said second conductive pads, such that said each piezoelectric pad covers its corresponding first conductive pad and protrudes from said first conductive pad, preferably, at least 2 mm larger. This configuration grants that there is no electrical contact between any part of the first and second conductive pads of each sensor without the need to arrange any additional dielectric layer between them. And an equivalent effect is obtained for their respective conductive lines.

Preferably, said pressure sensors are arranged in a grid, each of said first conductive lines is electrically connected in series to one of more of said first conductive pads, such that each first conductive pad is connected to only one first conductive line; and each of said second conductive lines is electrically connected in series to one of more of said second conductive pads, such that each second conductive pad is connected to only one second conductive line. Therefore, each first conductive line is connected in series as a so-called daisy chain of first conductive pads, and each second conductive line is connected in series as a daisy chain of second conductive pads. This arrangement minimizes the number of conductive lines that are required for obtaining the measures of each pressure sensor. For example, when reading a particular pressure sensor, it may be envisaged that the measure is taken form the first conductive line that is connected to the first conductive pad of said pressure sensor and the second conductive line that is connected to the second conductive pad of the pressure sensor. Each pressure sensor is connected only to a first conductive line and a second conductive line, thereby minimizing the required number of lines.

Preferably, for each pressure sensor, the first conductive line connected to its corresponding first conductive pad is perpendicular to the second conductive line connected to its corresponding second conductive pad. This way, the grid is formed by perpendicular conductive lines having a pressure sensor in the nodes, and more preferably, forming a matrix of pressure sensors. The perpendicular arrangement made of conductive lines and, in particular, when said conductive lines are made of a polymer having elastic capabilities, provides the film with a homogeneous patter resulting in a film that stretches and contracts in a more homogenous way.

The invention also refers to a method for manufacturing a pressure sensing film according to any of the embodiments discloses above, characterized in that it comprises the following steps:
a) arranging said substrate layer in a screen printer;
b) screen printing said first conductive layer over said substrate layer using an ink made of said first electrically conductive polymer;
c) screen printing said piezoelectric layer using an ink made of said piezoelectric polymer;
d) screen printing said second conductive layer using an ink made of said second electrically conductive polymer;
e) screen printing said third conductive layer using an ink made of said electrically conductive material;
f) screen printing said dielectric layer using an ink made of said elastic stretchable dielectric material, covering all the underlying layers; and
g) polarizing said piezoelectric layer.

The technical effects provided by the method have been already discussed previously in this document or are equivalent to those described for the pressure sensing film. Therefore, for the sake of brevity, they will not be repeated here.

Regarding the polarizing step g), it may be envisaged that, contrary to the standard polarization of piezoelectric elements, the field applied to the film is a wide field, that is, it affects more than one piezoelectric pad and, preferably, all the piezoelectric pads. This type of polarization diminishes the global manufacturing time, and the resulting polarization has been found to be enough to obtain accurate measurements from the pressure sensors. Preferably, the polarization is done by poling with 130 MV/m for 30 seconds and 500 milliseconds increasing ramp.

Preferably, the method further comprises an additional step of screen printing or spaying an adhesive layer under said substrate layer and/or over said substrate layer.

The invention also refers to a pressure analysis system comprising:
- a pressure sensing film according to any of the embodiments disclosed above;
- a multiplexing means, electrically connected to said third terminal ends of said third electrically conductive lines that are arranged in said connection part of said pressure sensing film, configured to receive electrical signals from said third electrical lines and selectively generate corresponding electrical signals for each pressure sensor;
- a measuring means, connected to said multiplexing means and configured for obtaining electrical measures of said electrical signals of each pressure sensor;
- a control means, configured for receiving and processing said electrical measures so as to obtain a corresponding pressure map; and
- a visualization means, configured to display said pressure map.

Different alternatives may be envisaged for the control means and visualization means. As non-limiting examples, a dedicated CPU and screen, a personal computer having specialized software and a display, a tablet or smartphone, or even a cloud computing infrastructure with results that can be visualized locally. Likewise, measuring means may be connected by wire or wirelessly to the multiplexing means. The advantages of the pressures sensing film have already been discussed above.

Preferably, said electrical signals are capacitance signals. Contrary to the usual practice wherein the piezoelectric elements are used for generating voltage measurements, it has been found that capacitive measurements provide better results in terms of accuracy and response time. A further advantage is that the linear range wherein the response is linear seems to be larger in the case wherein the capacitive response is measured. The particular arrangement of the pressure sensors of the pressure sensing film allows this type of measurements and signals and, therefore, contributes to the overall accuracy.

The invention also refers to the usage of a pressure sensing film according to any of the embodiments disclosed above for obtaining a pressure map of the thread of a user with a particular insole, comprising:
- arranging said insole inside a shoe;
- arranging said pressure sensing film over said insole;
- making the user to insert the foot inside the shoe and thread over a surface;
- obtaining a pressure map from measures of said pressure sensors, preferably capacitance measures.

The skilled person will understand that the first two steps may also be interchanged, so that first the pressure sensing film is arranged inside a shoe and then said insole is arranged over said pressure sensing film.

The pressure map may be then used by a podiatrist or another user of the system for validating the correct adaptation of the insole to the foot of the user.

Preferably, the method is done for the two feet of the user at the same time, wherein each foot is inserted in its corresponding shoe, having a corresponding insole and a corresponding pressure sensing film, thereby obtaining a pressure map of the thread of each foot.

The invention also refers to the usage of a pressure sensing film according to any of the embodiments disclosed above for obtaining a pressure map of a user mounting a particular saddle, comprising:
- arranging said saddle over a mount, in particular a horse;
- arranging said pressure sensing film over said saddle;
- making the user to mount said saddle;
- obtaining a pressure map from measures of said pressure sensors, preferably capacitance measures.

The skilled person will understand that the first two steps may also be interchanged, so that first the pressure sensing film is arranged over the mount and then said saddle is arranged over said pressure sensing film.

The invention also includes other detail features illustrated in the detailed description of some embodiments of the invention and in the accompanying figures.

### Brief description of the figures

Further advantages and features of the invention will become apparent from the following description, in which, without any limiting character, preferred embodiments of the invention are disclosed, in reference to the accompanying figures:
Fig. 1 is a schematic representation of a pressure sensing film according to the invention which is aimed for characterization of an insole of a shoe. The different pressure sensors have been identified with numbers that are exclusive for the identification of each sensor and do not correspond to the numerical references used in the document. Likewise, the terminals are also identified by exclusive numbers that are not related to the numerical references.
Fig. 2 is a detailed representation of the connection part and the sensing part of the pressure sensing film of Fig. 1.
Fig. 3 shows different stages of manufacturing of the pressure sensing film of Fig. 1.

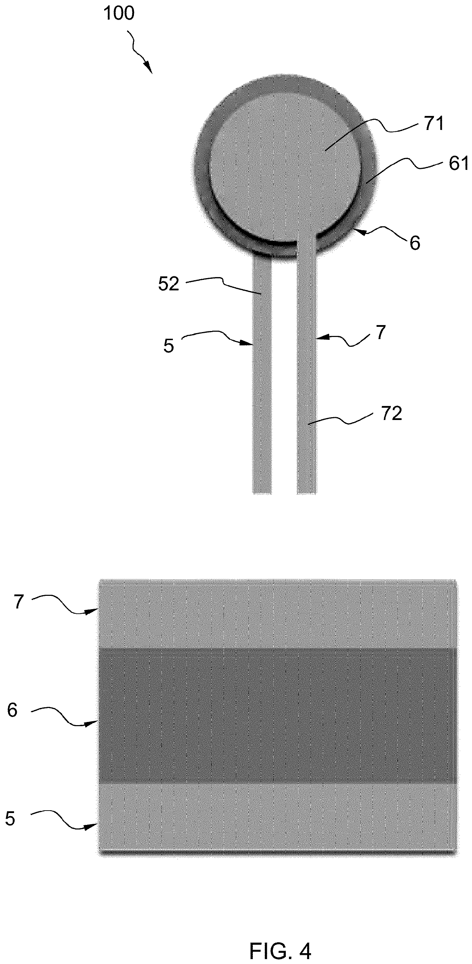

Fig. 4 is a schematic representation of the different layers forming a pressure sensor according to the invention. The upper drawing shows a top view of the capacitor-like sensor, and the bottom drawing represents a section of the different layers.

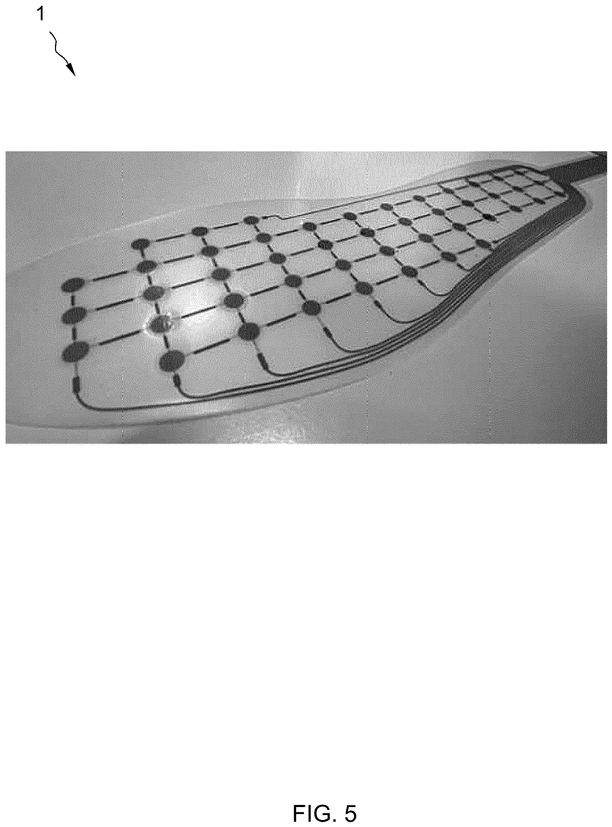

Fig. 5 is a real photograph of the pressure sensing film, showing a closeup of the sensing part viewed in perspective.

### Detailed description of embodiments of the invention

The figures show a pressure sensing film 1 according to a first embodiment the invention. In the first embodiment, the pressure sensing film 1 is aimed for obtaining a pressure map of the thread of a user with a particular insole. The pressure sensing film 1 has a sensing part 2 and a connection part 3. The sensing part 2 is configured for being positioned between a supporting surface and a part of a user in a usage position. In the case o the first embodiment, said supporting surface is an insole of a shoe and said part of the user is the sole of a foot of said user.

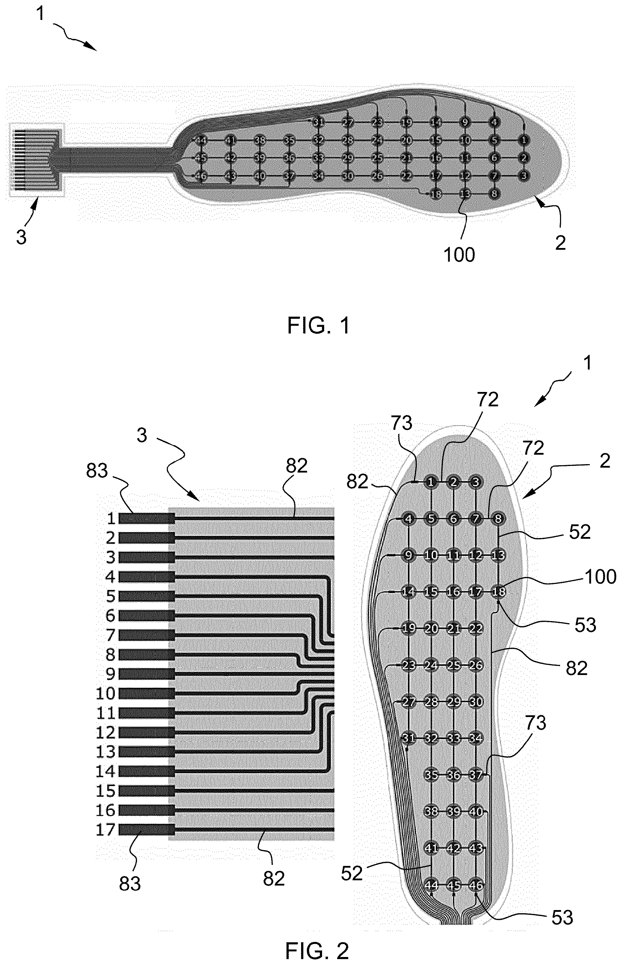

A plurality of pressure sensors 100 are formed in the sensing part 2. Each pressure sensor 100 having a capacitor-like structure, with a piezoelectric pad 61 arranged between two conductive pads 51, 71. In the schematic representations shown in Fig. 1 and Fig. 2, each of the pressure sensors 100 is identified with a number. These numbers are exclusively related to the identification of the pressure sensors 100 and not related to the numerical references used in this document.

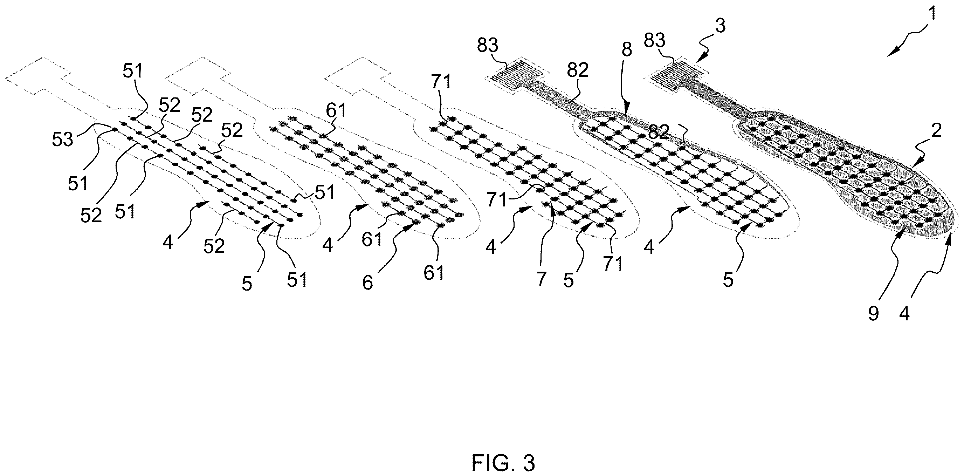

Fig. 3 shows different stages of the manufacture of the pressure sensing film 1. For the sake of clarity, only some exemplary elements are marked with numerical references in the figures. In the case of the first embodiment, each of the layers of the pressure sensing film 1 is formed by screen printing. From left to right, the first drawing shows that the pressure sensing film 1 is formed by a substrate layer which is made of an elastic stretchable material, in particular, thermoplastic polyurethane, TPU. The substrate layer 4 is arranged in a screen printer and receives a pre-treatment of 140 °C for 20 min. The screen printer then prints a first conductive layer 5 over said substrate layer 4 using an ink made of a first electrically conductive polymer, in particular, poly3,4-ethylenedioxythiophene polystyrene sulfonate, PEDOT:PSS.

The first conductive layer 5 has a plurality of first conductive pads 51 and at least one first conductive line 52, arranged in the sensing part 2. The figures shown five first conductive lines 52 and forty-six first conductive pads 51, but this numbers may be different depending, for example, on the size of the insole. Each first conductive pad 51 is connected to only one of the first conductive lines 52, however, other embodiments may be envisaged wherein one or more of the first conductive pads 51 is connected to mor than one first conductive line 52, depending on the type of interconnection desired for the pressure sensors 100. Each first conductive line 52 has a first terminal end 53. The first conductive layer is then cured with a temperature of 130°C for 10 minutes.

The second drawing of Fig. 3 shows the next layer that is formed over the previous ones. In particular, a piezoelectric layer 6 is printed using an ink made of a piezoelectric polymer. In the case of the first embodiment, said piezoelectric polymer is polyvinylidene fluoride-co-trifluoroethylene, PVDF-TrFE. The piezoelectric layer 6 has a plurality of piezoelectric pads 61, each arranged over one of the first conductive pads 51 of the first conductive layer 5. Therefore, in the example of the figures, forty-six piezoelectric pads 61 are formed. In this embodiment, the piezoelectric pads 61 are larger than the first conductive pads 51, such that said each piezoelectric pad 61 covers its corresponding first conductive pad 51 and protrudes around 2mm from said first conductive pad 51. The piezoelectric layer 6 is then cured at 140°C for 10 minutes.

The third drawing of Fig. 3 shows a second conductive layer 7 formed over the previous layers. Said second conductive layer 7 is also formed by screen printing using an ink made of an electrically conductive polymer which, in this embodiment, is the same that the one used in the first conductive layer, i.e., PEDOT:PSS.

As shown in Fig. 3. The second conductive layer 7 has a plurality of second conductive pads 71 and several conductive lines 72 arranged in said sensing part 2. Each second conductive line 72 has a second terminal end 73. Each second conductive pad 71 is formed over one of said piezoelectric pads 61, thereby creating the plurality of capacitor-like pressure sensors 100. The second conductive pads 71 are smaller than the corresponding piezoelectric pads 61, so that the underlying piezoelectric pad 61 protrudes around 2mm from the second conductive pad 71. In the examples of the figures, forty-six second conductive pads 71, and twelve conductive lines 72 are formed. Each second conductive pad 71 is connected to only one of the second conductive lines 72. As it can be seen in the figures, for example in Fig. 1 and Fig. 2, the pressure sensors 100 of the first embodiment are arranged in a grid: each of the first conductive lines 52 is electrically connected in series to one of more of the first conductive pads 51, such that each first conductive pad 51 is connected to only one first conductive line 52; and each of the second conductive lines 72 is electrically connected in series to one of more of the second conductive pads 71, such that each second conductive pad 71 is connected to only one second conductive line 72. In particular, for each pressure sensor 100, the first conductive line 52 and the second conductive line 72 connected thereof are perpendicular, and a matrix of pressure sensors 100 is formed. After the screen printing, the second conductive layer 7 is cured at 130°C for 10 minutes.

The fourth drawing of Fig. 3 shows a third conductive layer 8, arranged over at least part of said second conductive layer 7, part of the first conductive layer 5, and part of the substrate layer 4. The third conductive layer 8 is formed by screen printing using an ink made of said electrically conductive material, in particular, silver. Said third conductive layer 8 has a plurality of third electrically conductive lines 82 each having one third terminal end 83 at the connection part 3, and another end connected to one of the first terminal ends 53 or one of the second terminal ends 73. In the schematic representations shown in Fig. 1 and Fig. 2, each of the third terminal ends 83 is numbered with its own identification number that does not relate to the numerical references used in the documents, or the identification numbers of the pressure sensors 100, as it will be clear by using different font type/size/colour. In the example shown in the figures, there is seventeen third terminal ends 83, each connected to one of the five first terminal ends 53 or one of the twelve second terminal ends 73. For example, third terminal end 83 with ID 1 is connected to the second terminal end 73 near the pressure sensor 100 with ID 1, and third terminal end 83 with ID 17 is connected to the first terminal end 53 near the pressure sensor 100 with ID 18. The main part of several third electrically conductive lines 82 arranged over the sensing part 2 of the pressure sensing film 1 are arranged following one of the edges of the sensing part, between 3 and 10mm form said edge. The third conductive layer 8 is then cured at 120°C for 10 minutes.

The fifth and final drawing of Fig. 3 shows the formation of the top-level layer in the first embodiment of the invention, in particular, a dielectric layer 9 that is screen printed using an encapsulating ink (Dupont 8155) as an elastic stretchable dielectric material. The layer is arranged covering all the underlying layers, albeit not necessarily covering the totality of the underlaying layers, for example, the dielectric layer 9 does not cover the third terminal ends 83. The dielectric layer 9 is cured at 140°C for 10 minutes.

The piezoelectric layer 6 is then polarized by poling of the PVDF-TrFE with 130 MV/m for 30 seconds and 500 milliseconds increasing ramp.

The pressure sensing film 1 of the first embodiment may be used for obtaining a pressure map of the thread of a user with a particular insole by:
- arranging said insole inside a shoe;
- arranging said pressure sensing film 1 over said insole;
- making the user to insert the foot inside the shoe and thread over a surface;
- obtaining a pressure map from measures of said pressure sensors 100, preferably capacitance measures.

The process may be also done with the two feet at the same time, each using its own insole, shoe and pressure sensing film 1.

A pressure analysis system for performing the measurement comprises:
- a pressure sensing film 1, usually being disposable;
- a multiplexing means, electrically connected to said third terminal ends 83 of said third electrically conductive lines 82 that are arranged in said connection part 3 of said pressure sensing film 1, configured to receive electrical signals from said third electrically conductive lines 82 and selectively generate corresponding electrical signals for each pressure sensor 100;
- a measuring means, connected to said multiplexing means and configured for obtaining electrical measures of said electrical signals of each pressure sensor 100;
- a control means, configured for receiving and processing said electrical measures so as to obtain a corresponding pressure map; and
- a visualization means, for example, a screen, configured to display said pressure map.

In the first embodiment, said electrical signals are capacitance signals.

Other embodiments of the pressure sensing film 1 according to the invention are disclosed hereinafter. These embodiments share most of the features disclosed in the first embodiment above. Therefore, only the differentiating features will be described in detail. For the sake of brevity, common features shared with the first embodiment disclosed above will not be described again hereinbelow.

In a second embodiment, not shown in the figures, after curing the dielectric layer 9 and before the polarization step, an adhesive layer is screen printed on the reverse of the substrate layer 4, not covering the active area of the pressure sensors 100. The adhesive layer is cured with UV light for 3 seconds.

In a third embodiment, not shown in the figures and derived from the first embodiment; after curing the dielectric layer 9 and before the polarization step, an adhesive layer is screen printed over the other layers not covering the terminals and the active areas. The adhesive layer is cured with UV light for 3 seconds.

In a fourth embodiment, not shown in the figures, the supporting surface is a saddle, for example, a horse saddle, and the part of the user are the buttocks of said user. In this embodiment, the pressure sensing film 1 is used for obtaining a pressure map of the user mounting a particular saddle, by following the steps of:
- arranging said saddle over a mount, in particular a horse;
- arranging said pressure sensing film 1 over said saddle;
- making the user to mount said saddle;
- obtaining a pressure map from measures of said pressure sensors 100, preferably capacitance measures.

## Claims

1. Pressure sensing film (1) having a sensing part (2) and a connection part (3), said sensing part (2) configured for being positioned between a supporting surface and a part of a user in a usage position, **characterized in that** said pressure sensing film (1) comprises:
- a substrate layer (4), made of an elastic stretchable material;
- a first conductive layer (5), arranged over said substrate layer (4);
- a piezoelectric layer (6), arranged over said first conductive layer (5);
- a second conductive layer (7), arranged over said piezoelectric layer (6);
- a third conductive layer (8), arranged over at least part of said second conductive layer (7); and
- a dielectric layer (9), made of an elastic stretchable dielectric material, arranged over said third conductive layer (8) and covering all the underlying layers;
wherein said first conductive layer (5) comprises a plurality of first conductive pads (51) and one or more first conductive lines (52), all made of a first electrically conductive polymer, and arranged in said sensing part (2), such that each first conductive pad (51) is connected to at least one of said first conductive lines (52), each first conductive line (52) having a first terminal end (53);
said piezoelectric layer (6) comprises a plurality of piezoelectric pads (61) made of a piezoelectric polymer; each piezoelectric pad (61) arranged over one of said first conductive pads (51) in said sensing part (2);
said second conductive layer (7) comprises a plurality of second conductive pads (71) and one or more second conductive lines (72), all made of a second electrically conductive polymer, and arranged in said sensing part (2), such that each second conductive pad (71) is connected to at least one of said second conductive lines (72), each second conductive line (72) having a second terminal end (73), and wherein each second conductive pad (71) is arranged over one of said piezoelectric pad (61), so that a plurality of pressure sensors (100) having a capacitor-like structure is formed, each pressure sensor (100) having a piezoelectric pad (61) arranged between a first conductive pad (51) and a second conductive pad (71); and
said third conductive layer (8) comprises a plurality of third electrically conductive lines (82) made of an electrically conductive material, each third electrically conductive line (82) having one third terminal end (83) at said connection part (3) and another end connected to one of said first terminal ends (53) or one of said second terminal ends (73), said dielectric layer (9) not covering said third terminal ends (83).

2. Pressure sensing film (1) according to claim 1, wherein said elastic stretchable material of said substrate layer (4) is thermoplastic polyurethane, TPU.

3. Pressure sensing film (1) according to any of the claims 1 or 2, wherein said first conductive polymer is the same that said second conductive polymer, preferably poly(3,4-ethylenedioxythiophene) polystyrene sulfonate, PEDOT:PSS; and preferably wherein said piezoelectric polymer is poly(vinylidene fluoride-co-trifluoroethylene), PVDF-TrFE.

4. Pressure sensing film (1) according to any of the claims 1 to 3, wherein said electrically conductive material of said third conductive layer (8) is a metal, preferably silver, wherein each of said third electrically conductive lines (82) is preferably arranged following one of the edges of said sensing part (2), preferably between 3 and 10mm from said edge.

5. Pressure sensing film (1) according to any of the claims 1 to 4, wherein at least one of said first conductive layer (5), said piezoelectric layer (6), said second conductive layer (7), said third conductive layer (8) and said dielectric layer (9), and preferably all said layers, are formed by screen printing.

6. Pressure sensing film (1) according to any of the claims 1 to 5, further comprising an adhesive layer arranged under said substrate layer (4), such that in said usage position, said adhesive layer is in contact with said supporting surface, said adhesive layer preferably formed by screen printing or by spraying.

7. Pressure sensing film (1) according to any of the claims 1 to 6, wherein said piezoelectric pads (61) are larger than said first conductive pads (51) and said second conductive pads (71), such that said each piezoelectric pad (61) covers its corresponding first conductive pad (51) and protrudes from said first conductive pad (51).

8. Pressure sensing film (1) according to any of the claims 1 to 7, wherein said pressure sensors (100) are arranged in a grid, each of said first conductive lines (52) is electrically connected in series to one of more of said first conductive pads (51), such that each first conductive pad (51) is connected to only one first conductive line (52); and each of said second conductive lines (72) is electrically connected in series to one of more of said second conductive pads (71), such that each second conductive pad (71) is connected to only one second conductive line (72), preferably, wherein, for each pressure sensor (100), the first conductive line (52) connected to its corresponding first conductive pad (51) is perpendicular to the second conductive line (72) connected to its corresponding second conductive pad (71), more preferably, forming a matrix of pressure sensors (100).

9. Pressure sensing film (1) according to any of the claims 1 to 8, wherein:
- said supporting surface is an insole of a shoe and said part of the user is the sole of a foot of said user; or
- said supporting surface is a saddle and said part of the user are the buttocks of said user.

10. Method for manufacturing a pressure sensing film (1) according to any of the claims 1 to 9, **characterized in that** it comprises the following steps:
a) arranging said substrate layer (4) in a screen printer;
b) screen printing said first conductive layer (5) over said substrate layer (4) using an ink made of said first electrically conductive polymer;
c) screen printing said piezoelectric layer (6) using an ink made of said piezoelectric polymer;
d) screen printing said second conductive layer (7) using an ink made of said second electrically conductive polymer;
e) screen printing said third conductive layer (8) using an ink made of said electrically conductive material;
f) screen printing said dielectric layer (9) using an ink made of said elastic stretchable dielectric material, covering all the underlying layers; and
g) polarizing said piezoelectric layer (6).

11. Method for manufacturing a pressure sensing film (1) according to claim 10, wherein the method further comprises an additional step of screen printing or spaying an adhesive layer under said substrate layer (4) and/or over said substrate layer (4).

12. Pressure analysis system comprising:
- a pressure sensing film (1) according to any of the claims 1 to 9;
- a multiplexing means, electrically connected to said third terminal ends (83) of said third electrically conductive lines (82) that are arranged in said connection part (3) of said pressure sensing film (1), configured to receive electrical signals from said third electrically conductive lines (82) and selectively generate corresponding electrical signals for each pressure sensor (100);
- a measuring means, connected to said multiplexing means and configured for obtaining electrical measures of said electrical signals of each pressure sensor (100);
- a control means, configured for receiving and processing said electrical measures so as to obtain a corresponding pressure map; and
- a visualization means, configured to display said pressure map.

13. Pressure analysis system according to claim 12, wherein said electrical signals are capacitance signals.

14. Usage of a pressure sensing film (1) according to any of the claims 1 to 9 for obtaining a pressure map of the thread of a user with a particular insole, comprising:
- arranging said insole inside of a shoe;
- arranging said pressure sensing film (1) over said insole;
- making the user to insert the foot inside the shoe and thread over a surface;
- obtaining a pressure map from measures of said pressure sensors (100), preferably capacitance measures.

15. Usage of a pressure sensing film (1) according to any of the claims 1 to 11 for obtaining a pressure map of a user mounting a particular saddle, comprising:
- arranging said saddle over a mount, in particular a horse;
- arranging said pressure sensing film (1) over said saddle;
- making the user to mount said saddle;
- obtaining a pressure map from measures of said pressure sensors (100), preferably capacitance measures.
